# EUROPEAN PATENT SPECIFICATION

(11) **EP 4 377 668 B1**
(45) Date of publication and mention of the grant of the patent: **28.10.2026**
(21) Application number: 22854466.4
(22) Date of filing: 01.08.2022
(51) Int. Cl.: G01N 3/12, G01N 33/24

(54) **TRIAXIAL TESTING APPARATUS WITH MULTIPLE MEASURING CELLS**
DREIACHSIGE TESTVORRICHTUNG MIT MEHREREN MESSZELLEN
APPAREIL D'ESSAI TRIAXIAL AYANT DE MULTIPLES CELLULES DE MESURE

(30) Priority: 31.07.2021 HU 2100283
(43) Date of publication of application: 05.06.2024
(73) Proprietor: Széchenyi István Egyetem, 9026 Gyor (HU)
(72) Inventor: HUDACSEK, Péter, 2016 Leányfalu (HU)
(74) Representative: Molnar, Istvan
(86) International application number: PCT/HU2022/050059
(87) International publication number: WO 2023/037133

(56) References cited:
- CN-A- 109 253 925
- CN-U- 201 600 308
- US-A1- 2017 150 015

## Description

The present invention relates to the study of bulk properties of samples. More specifically, the subject matter of the present invention is a triaxial testing apparatus for the load testing of samples, primarily of soils and other porous and highly viscous masses of similar structure.

When designing and constructing structures and buildings, it is extremely important from a geotechnical and structural point of view to know the strength, bearing capacity and leakage properties of the soils at the construction site. These can be determined using so-called triaxial (three-axis) measuring cells, by means of a triaxial testing apparatus, with the soil samples being placed in the measuring cell, usually one after the other, and then measured.

The research paper by S. E. Salazar entitled "Development of an Internal Camera-Based Volume Determination System for Triaxial Testing", published in December 2017 (University of Arkansas, Fayetteville, USA; Theses and Dissertations) describes a camera-based volume determination system for triaxial testing of saturated and unsaturated soil samples in a single soil measuring cell. The cell is equipped with cameras mounted on rotating arms to measure the volume and deformation of the soil sample. A three-dimensional model of the sample is mapped using photogrammetry. The system contains silicone oil to provide hydrostatic load in the cell, in which the cameras are fully immersed during operation.

The scientific paper by D. Solav et al. entitled "MultiDIC: An Open-Source Toolbox for Multi-View 3D Digital Image Correlation", published on June 4, 2018, provides an introduction to the measurement principle of Spatial Digital Image Correlation (3D-DIC) and its practical implementation. The measurement is based on the usage of cameras symmetrically arranged in 360° around the object to be studied, and the presented technical solution consists of twelve cameras, the object surface images are captured by stereoscopic imaging with adjacent camera pairs. The method involves tracking/measuring the changes of each point in a dot pattern projected onto the object under study relative to a reference configuration, and the pieces of information related to the deformation of the object during the measurement is determined from this information. Said paper also discusses in detail the mathematical background applied for the evaluation of the captured images.

Chinese Patent Application No. 109253925 related to a triple temperature control unsaturated soil triaxial test system. The system comprises a loading platform, at least two parallel triaxial cells, a pressure control system, a temperature control system and a data measurement and acquisition system. Each triaxial cell comprises a cell base, a cell wall and a cell top cover; and an argil plate, a sample, a porous stone and a sample cap are sequentially arranged above the cell base from bottom to top. The pressure control system and the temperature control system are in circuit connection with the data measurement and acquisition system, wherein the pressure control system comprises a water pressure system, an air pressure system and a confining pressure system. The temperature of each cell in the temperature control system is uniformly controlled; the water pressure of each cell in the water pressure system is controlled uniformly; the air pressure of each cell in the air pressure system is independently controlled; and the confining pressure of each cell in the confining pressure system is independently controlled.

US Patent No. 10,542,195 B2 discusses possible embodiments of the above camera-based volume determination system. Said document examines whether gas (air) can be used in the measuring cell to exert the hydrostatic load. Due to improper optical properties of air and the technical parameters of the instruments used, in the volume determination system itself silicone oil is used. Mineral oil can also be used instead of silicone oil, which also avoids short circuits caused by direct contact between the electronics and fluid, while the mineral oil has adequate optical properties. Depending on the size of the soil sample, the position and number of cameras can be varied to ensure adequate coverage of the sample area. The camera system immersed in oil can be used up to a cell pressure of 1034 kPa.

Chinese Patent Application No. 112033800 A describes a triaxial testing method wherein the testing cameras are located outside the measuring cell. The method makes use of digital image processing technology to measure dynamic loading and unevenness of the entire surface. The testing apparatus consists of three high-speed industrial cameras and a modified triaxial compression chamber. The application basically details the alignment of the three camera images, the correction of errors that occur, and the processing of the deformation of the soil sample studied.

US Patent No. 10,564,079 B2 discloses a measuring cell, system and method wherein a hydrostatic load on a soil sample is generated by a high pressure gas (air). A closed measuring cell containing the soil sample is arranged in a pressure chamber and then the pressure chamber is filled with hydraulic oil. High pressure air is then fed into the measuring cell. The pressure differential between the measuring cell and the pressure chamber is compensated by a pressure control in such a way that the hydraulic oil in the pressure chamber can flow into the measuring cell through said pressure control, but the oil cannot reach the height of the soil sample arranged inside. The hydraulic oil prevents leakage of the high pressure gas, facilitates the determination of the rate of change of volume and compensates the pressure of the gas on the sidewall.

Chinese Utility Model Application No. 201600308 U teaches a triaxial testing apparatus of high-efficiency for simultaneous testing of multiple soil samples. Each individual soil sample studied is arranged in a separate pressure chamber, wherein the pressure chambers are served by the same pressure maintaining and data processing system, thereby allowing a more efficient utilization of the devices used for the study. As each pressure unit is located on a common overhead bridge, the samples can be tested in parallel. Measurements are performed by the testing apparatus by making use of sensors and a computer-based processing means.

The present invention aims to eliminate or at least mitigate the drawbacks of the above solutions.

A further object is to develop an improved triaxial testing apparatus and its operating procedure, which is capable of integrating and handling several triaxial measuring cells simultaneously, thereby allowing simultaneous measurement of multiple test specimens.

A yet further object is to achieve a triaxial testing apparatus wherein the feed rate of the test specimens in each individual measuring cell can be controlled independently with a required accuracy.

The test specimens in the measuring cells may be different soil samples, where appropriate, so that the improved triaxial testing apparatus according to the invention can provide soil mechanics laboratory testing services that meet all standards in a reasonably short turnaround time and at a reasonable price. However, the test specimens may also be highly viscous, high consistency masses, such as fresh concrete of different qualities, or other similar substances requiring strength testing. The measuring cells are either conventional frame-based pressure cells or Bishop-Wesley triaxial cells. The measuring cells used in the testing apparatus can be equipped with various accessories suitable for other or special measurements. Hence, the apparatus can be used for simultaneous measurements of deformation, compression characteristics, and permeability in addition to the conventional tests for determining shear strength parameters. Due to the construction, the improved triaxial testing apparatus is also suitable for performing high-precision stress field and slow cyclic tests in its basic configuration and, with the use of special accessories, unsaturated soils tests.

The essence of the improved triaxial testing apparatus according to the invention is that the measuring cells are arranged in a single block in the apparatus, and the control modules (preferably three in number) are mounted in the rear or bottom wall of the testing apparatus. Furthermore, the connection between the control modules and the measuring cells is provided by a hydraulic "multiplexer" unit, which eliminates the changes in load stresses, pore water pressure and specimen volume of the test specimens in the measuring cells during the changes of control between the measuring cells, which reduce the accuracy of the measurement and the reliability of the control. This is achieved by (i) disconnecting the previous measuring volume to be pressure controlled from the control side prior to the change of control, (ii) equalizing the pressures present on the side of the next measuring volume to be pressure controlled and on the side of the control unit by changing the latter along with very accurately measuring said pressures, and then (iii) connecting the control side to the next measuring volume to be pressure controlled. This procedure can be used for both hydraulic and pneumatic systems. In the improved triaxial testing apparatus, central pressure and displacement control (measuring cell, pore water, axial strain) systems are used.

Preferably, the measuring cells are made of fibre-reinforced material, are designed as lightweight and sufficiently safe pressure vessels, albeit with non-transparent walls, in which the cell pressure on the test specimen is provided by gas (compressed air) rather than liquid, and which will fail in a controlled, nonbrittle, non-explosive manner in the event of sufficiently high overpressures. The control of the cell pressures to an accuracy of 1 kPa is provided by a gas-saving, "on-demand" action valve system from a central cylinder pressure source. The valve system includes a group of safety valves to protect the cells from overpressure and a hydraulic control system which, in addition to providing basic control functions, is capable of disconnecting the gas supply to the measuring cells in a fail-safe mode and, if necessary, generates an alarm in the event of a sudden change in pressure (e.g. during testing and/or in the event of a diaphragm rupture due to activation of the safety relief valve or a rupture point activation).

In addition, in each measuring cell, the hydrostatic load is provided by the central "multiplexer" unit using gas, preferably (compressed) air, i.e. by gas pressure, instead of liquid (water or various oils); however, the cells are also designed in such a way that they can be used for measurements performed by the traditional "flooding" technique.

In the case of hydrostatic load exerted by air, the deformation of the test specimen in the measurement is measured by means of an optical measurement performed by a multi- or mobile camera arranged inside the measuring cell using stereo 3D DIC (digital image correlation) that is capable of determining the spatial displacement vectors of up to several thousand surface points of the test specimen with an accuracy of at least 1 pm. To meet high accuracy requirements, in the image processing/evaluation calculation procedure, a fast Fourier transform (FFT) based correlation is used, for 2D systems, a direct correlation calculation procedure based on a continuous window shift interpolation (CWS) technique used to reduce "peak-locking" is applied. This technique allows the analysis of the volume change and the total deformation field of the sample. Moreover, this technique also eliminates the need for the double-cell volume change measurements currently applied for unsaturated soils, as well as the Hall-effect or sample-mounted linear variable differential transformer (LVDT) based deformation measurements that are used for high-precision testing.

Tests with triaxial measuring cells are tests that take for relatively long time. During a full test, which can take from a few hours to several months, the pore volume typically changes by only a few cm³ and, even for a high resolution requirement (around 1 mm³ ), this requires very little intervention by the pump which control the change in the pore water pressure and volume. However, the cost and size of such precision pumps is significant. By reducing the number of pumps used in the improved triaxial testing apparatus according to the invention, significant space and cost savings are achieved that results in a compact embodiment of the apparatus. Particularly, in the triaxial testing apparatus according to the invention, pore water pressure control is provided alone by a central stepper motor pump connected to the lower end and the upper end of each specimen by means of routing/switching mechanisms (valve blocks) for pressure control and volume change measurement of all specimens. The high precision stepper motor driven linear actuator pump applied here is capable of volume change control with an accuracy of 1 mm³ and pressure measurement/control with an accuracy of 0.1 kPa alone for each single measuring cell over the full operating pressure range.

The objective to accomplish the improved triaxial testing apparatus is achieved by developing the apparatus according to claim 1 . Possible preferred exemplary embodiments of the testing apparatus according to the invention are defined in claims 2 to 5.

In what follows, the invention is described below in detail with reference to the accompanying drawing, wherein
- Figure 1A is a schematic perspective view of a triaxial testing apparatus according to the invention;
- Figure 1 B is an enlarged view of the apparatus illustrated in Figure 1A with some of the measuring cells integrated in the apparatus;
- Figures 2A and 2B are schematic block diagrams of the pressure control circuitries in the form of separate bottom and top pressure controls, respectively, for controlling the pore water pressure in each measuring cell in a preferred exemplary embodiment of the triaxial testing apparatus according to the invention with six measuring cells;
- Figure 3 is a flowchart presenting the operation of the pressure control circuitries illustrated in Figures 2A and 2B;
- Figure 4 illustrates a schematic block diagram for the pressure control of n measuring cells (n integer number) integrated in the triaxial testing apparatus according to the invention;
- Figure 5 illustrates a schematic block diagram for the cell pressure control circuitry of a triaxial testing apparatus according to the invention with six measuring cells;
- Figure 6 is a flowchart presenting the operation of the cell pressure control circuitry shown in Figure 5; - Figure 7 shows a schematic block diagram of a preferred exemplary embodiment of a hydraulic system used for the displacement control required to generate the deviator voltage in the triaxial testing apparatus according to the invention; and
- Figure 8 is a flowchart presenting the operation of the hydraulic system illustrated in Figure 7.

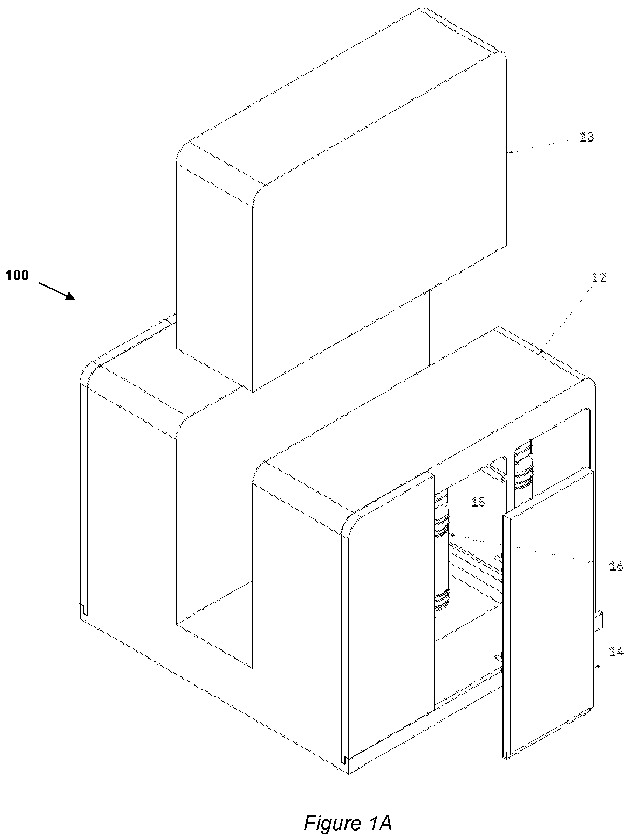

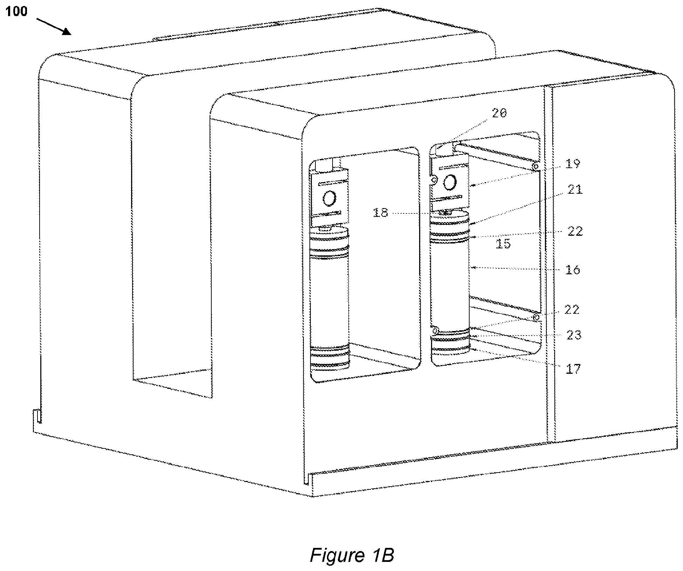

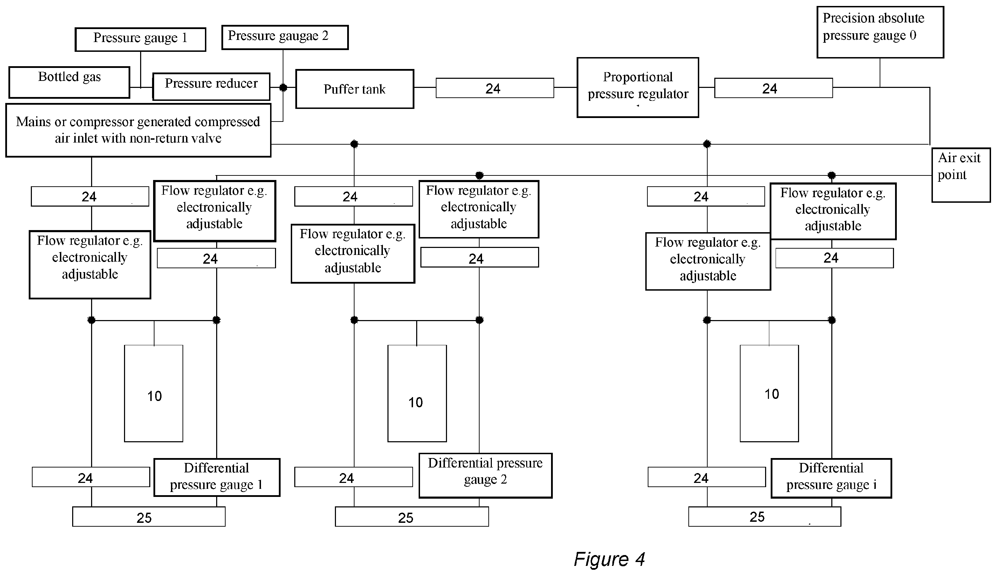

The triaxial testing apparatus 100 according to the invention, illustrated in Figures 1A and 1 B, is preferably a unit with overall dimensions of 100*100*85 cm, and constructed of shell elements 12 regarding its outer casing 14, wherein said casing 14 forms a solid figure in its appearance, and wherein a single central control module 13 serves, through a central hydraulic multiplexer unit, n pieces of measuring cells 10 being arranged in a test space 15, preferably within the casing 14 (see Figure 4). Each measuring cell 10 is used to perform triaxial compressive strength, stress field tests of cylindrical soil samples up to a sample size of D=100, L=200 mm (diameter, length). The measuring cells 10 are either conventional frame-based pressure cells or Bishop-Wesley triaxial cells. The number of said measuring cells 10 is preferably between a minimum of two and a maximum of twelve, and particularly preferably six. It should be here noted that the usage of six cells 10 is particularly advantageous because of the compactness of the testing apparatus 100 in this way. The technical modifications in the construction of the testing apparatus 100 required due to the number of the measuring cells 10 used are obvious to a person skilled in the art. In addition, with the use of special accessories, the testing apparatus 100 can also be used for conventional load cell five-dimensional (i.e. odometric) tests with constant deformation rate and stackable cells, as is also obvious to a skilled person in the art.

The central control module 13 (the multiplexer unit) is connected to a base of the measuring cells 10 by a single combined electrical, liquid/gas connector, and the control module 13 can be detached from the measuring cells 10 for maintenance, repair and upgrade in one single movement.

The deviator voltage is controlled by a hydraulic working cylinder arranged in the testing apparatus 100, the cell pressure is generated preferentially by air, and the pore water pressure is controlled by two single-piston pumps 5, 8 each having a geared stepper motor drive with independently operable motors. Here, one of the water chambers of said pumps is connected to the test specimen 7 under study through a filter stone 22 arranged on the lower plane of the test specimen 7 and the other of the water chambers is connected to the test specimen 7 under study through a further filter stone 22 arranged on the upper plane of said test specimen 7.

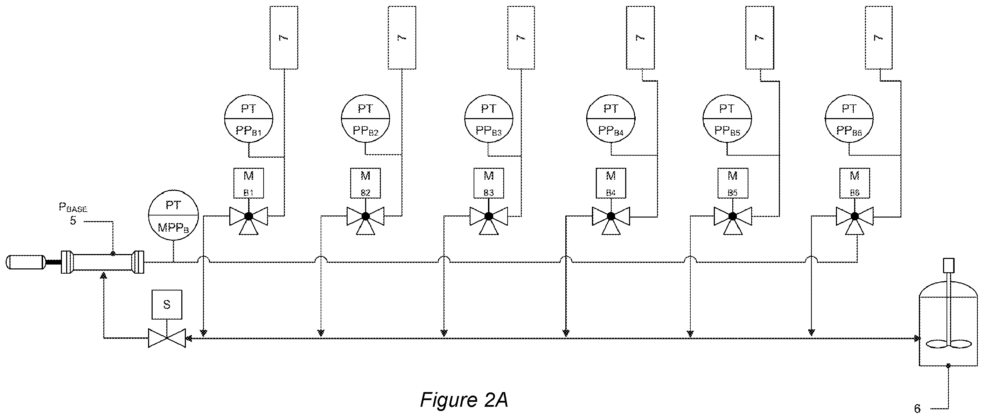

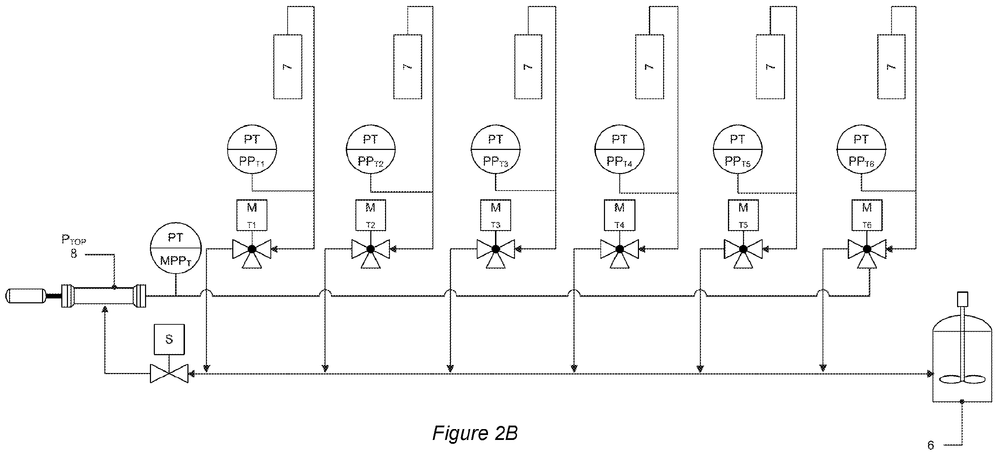

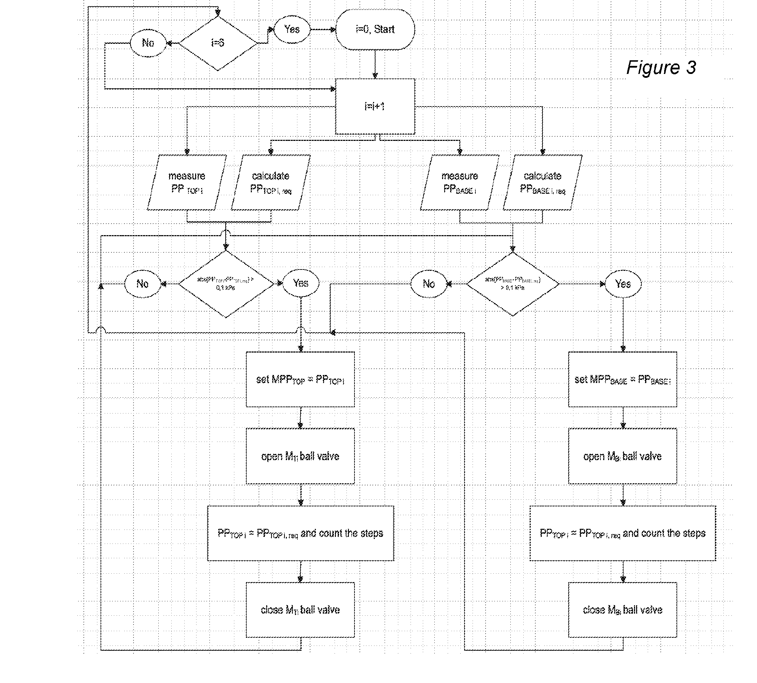

Figures 2A and 2B, and Figure 3 illustrate the pore water pressure control in a preferred embodiment of the triaxial testing apparatus of the invention realized preferably with six measuring cells.

A flexible pore water pipe runs freely in each measuring cell 10 from a top load plate to a point of exit from the respective cell, said pipe has a sufficiently large wall thickness and small internal diameter so as a change in cell pressure does not induce a change in the internal volume of said pipe.

Division of the pore water pressure control system is designed in such a way that only elements that do not change the internal volume of the water chambers when they are switched are included. For example, instead of a solenoid valve, a right angle ball valve is preferably used.

According to the pore water pressure control applied, if a lower or upper pressure gauge associated with a given test specimen 7 shows a difference larger than a set permissible deviation from the required pressure at a certain moment, then, with the valves closed, a pressure equal to that of the actual pressure of the pore space to be controlled is created on a pump 5, 8 (control) side of the valve. After the pressure has been established, the water chambers on the regulating and controlled sides being at the same pressure are opened up by means of a valve S inducing no volume changes, and then a measured amount of water is introduced into or discharged from the pore space by the geared stepper motor driven pump 5, 8 in steps with a resolution of at least 1 mm³ , along with counting the number of steps performed with the stepper motor. The measurement of water volume through step counting is known from the state of the art. However, said water volume measurement can also be performed with a hydraulic system that also incorporates a high-precision bidirectional differential pressure gauge with a small measuring range, whereby the pressure differential between the two sides can be set within a range of 10-20 Pa. This way, it is guaranteed that the same pressure prevails on both sides of the pressure gauge while it is inactive, and thus its null position can be calibrated before each active measurement, regardless of the temperature, line pressure, age of the gauge, etc..

Using the separate bottom and top pressure controls shown in Figures 2A and 2B in the triaxial testing apparatus 100 according to the invention, background pressure constant yield permeability tests can be also performed in the measuring cells 10.

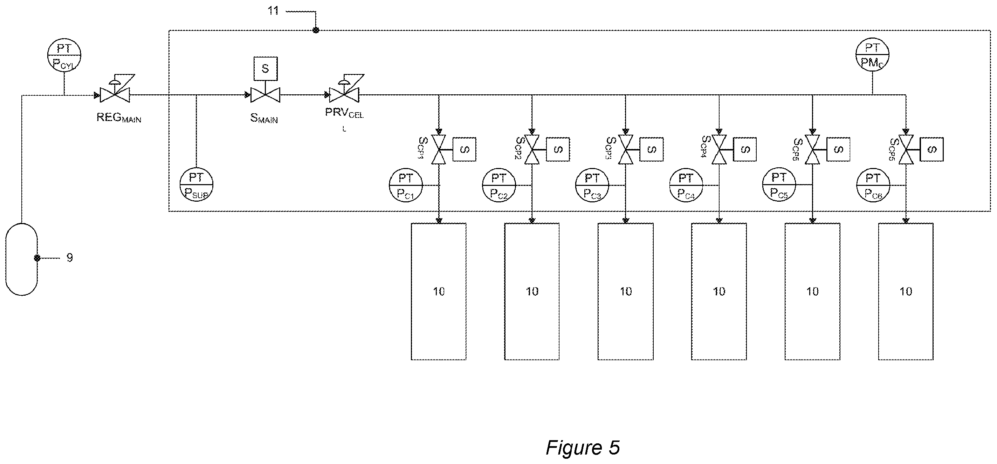

Referring now to Figs. 4 to 6, a central pressure control of the hydrostatic load provided by gas in the measuring cells 10 of the triaxial testing apparatus 100 according to the invention is described briefly for a general case of n measuring cells 10 (see Fig. 4) and for a preferred embodiment of the testing apparatus 100 according to the invention comprising six measuring cells 10 (see Figs. 5 to 6).

Controlling hydrostatic load with a gas is a simpler solution than the conventional liquid backing (water or silicone oil) ones, which facilitates the use of sensors mounted on the sample or used to monitor it, e.g. optical strain gauges, LVDTs, strain gauges, local deformation gauges, etc., while in this way said devices do not need to be protected against the ingress of high pressure fluids and, in the case of optical measuring devices, transparency and refractive index compatibility are not relevant issues either. Due to the higher mechanical energy (gas under pressure) stored in the measuring cells 10, instead of the conventional transparent polycarbonate or glass-walled measuring cells 10 allowing direct observation, measuring cells 10 with a controlled failure mechanism or with sufficient safety against failure, made of a sufficiently tough material, are used, and sample observation is provided by one or more miniature on-board cameras based on CCD, CMOS or other systems placed in the measuring cells 10.

The control process involves supplying air to the triaxial testing apparatus 100 from a mechanically or electronically operated buffer tank with a pressure reduced to a slightly higher value than a pressure prevailing in the measuring cell 10 to be operated at the highest pressure amongst the measuring cells 10 used in parallel, from a pressure source 9 installed (e.g. a local compressor, mains compressed air, cylinder compressed inert gas or other).

In the conventional process, if the pressure control is achieved with an air-water interface ("bladder cell") and a mechanical or proportional valve solution, there is a continuous air loss due to the continuous operation of the precision control elements. In the triaxial testing apparatus 100 according to the invention, the gas consumption of the system is minimized, based on the perception that, apart from the filling in the slow tests which can be even done by an external compressor - and independently of the control system - gas is only fed or discharged on demand.

The essence of the control system is that the pressure is continuously measured in the measuring cells 10 and, if the pressure falls below a preset value, the pressure is maintained at a constant value by injecting or releasing support gas into or from said measuring cells 10.

Pressure measurement can be performed with high-precision, high-range absolute pressure gauges per measuring cells 10 or with low range differential pressure gauges per measuring cells 10, which measure the pressure difference between the measuring cells 10 and a respective small reference tank 25 associated with each of the measuring cells 10. In the latter case, it is sufficient to use a single high-precision, high-range pressure gauge in the complete control system, which is connected to the measuring 10 cell that requires intervention when an initial cell pressure setting takes place, or the gas temperature in the reference tanks 25 changes, or the cell pressure changes due to the testing routine in such a way that the cell pressure change approaches the measuring range of said differential pressure gauges. When the cell pressure is adjusted by the central high-precision controller, the measuring cell 10 and its respective reference tank 25 gets into communication with each other for the duration of the adjustment.

To protect said differential pressure gauges, when a certain percentage of their measuring range is reached, the pressure chamber of the measuring cell 10 and the respective reference tank 25 is opened up by a solenoid valve 24. To increase the pressure, the measuring cells 10 are connected to a common main branch, which is adjusted by a proportional valve to a pressure slightly above the pressure of the measuring cell 10 to be just controlled, through a solenoid valve per measurement cells 10 that is either regulated by a throttle valve or reduced fixedly with a small flow cross-section and connected in series with said throttle valve, or through any other on-off valve.

To reduce the pressure, the measuring cells 10 are connected to a common pressure relief branch, open to the atmospheric pressure, by one or more branches per measuring cells 10, wherein some of the branches are either regulated each by a throttle valve or reduced fixedly with a small flow cross-section, or might be unreduced. For said pressure reduction, solenoid valves or any other on- off valves operated on the branches concerned are used.

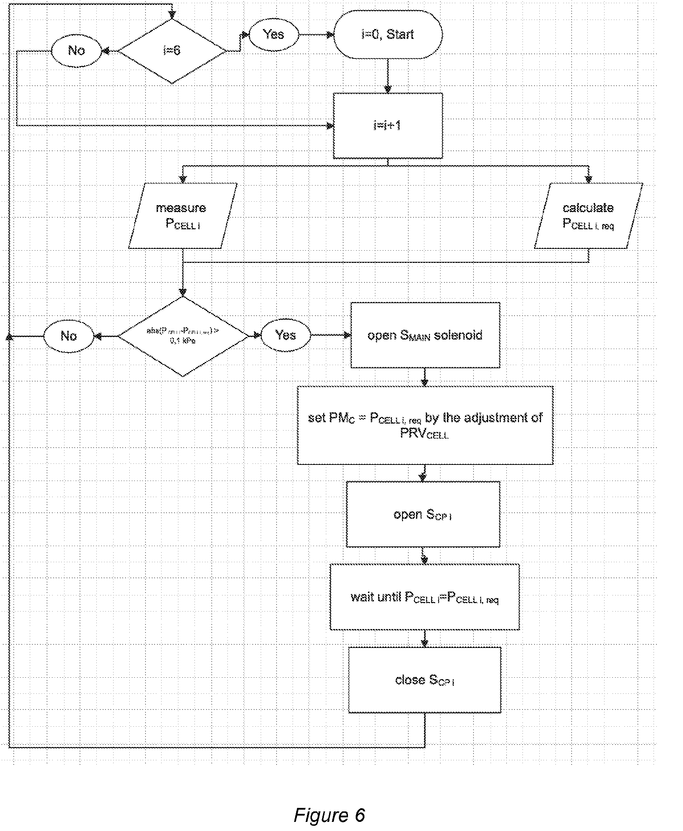

In the cases illustrated in Figures 5 and 6, air is used to create cell pressure, whereas traditionally cell pressure is adjusted with water. The advantage of applying pressure directly with air is that to measure small deformations and volume changes of a sample, in-cell force transducers of conventional design and conventional optical deformation sensing systems consisting of cameras can be used. To measure local deformation, which is intended for measuring deformation with an accuracy required by stress field tests, difficult-to-install "local deformation meters" with waterproof enclosures were used previously. The measurement of the change in volume of the soil sample forming the test specimen based on the change in volume of water in the cell is of importance for unsaturated soils, since in such cases the change in volume of the sample is not equal to the amount of water having been displaced from the sample. A change in volume of the sample can thus be measured by optical volumetric change measurement without the use of special double-walled measuring cells.

In this case the basic principle for an accurate and air-efficient control of the cell pressure is that the proportional valve responsible for air consumption only receives air on its supply side if the cell pressure in a cell differs from a value specified instantaneously by more than a preset tolerance limit. In such a case the position of the proportional valve is preset with a signal level corresponding to the pressure value to be achieved. Then, the valve on the inlet side is opened, the value of the adjusted pressure is checked and, if necessary, the position of the proportional valve is adjusted. Once the pressure got stabilized, the solenoid valve of the space to be adjusted is opened and it is allowed for the cell pressure to get stabilized. After the cell pressure got stabilized, the cell is closed and, if there is no further control action to be performed, the inlet valve of the proportional valve is also closed.

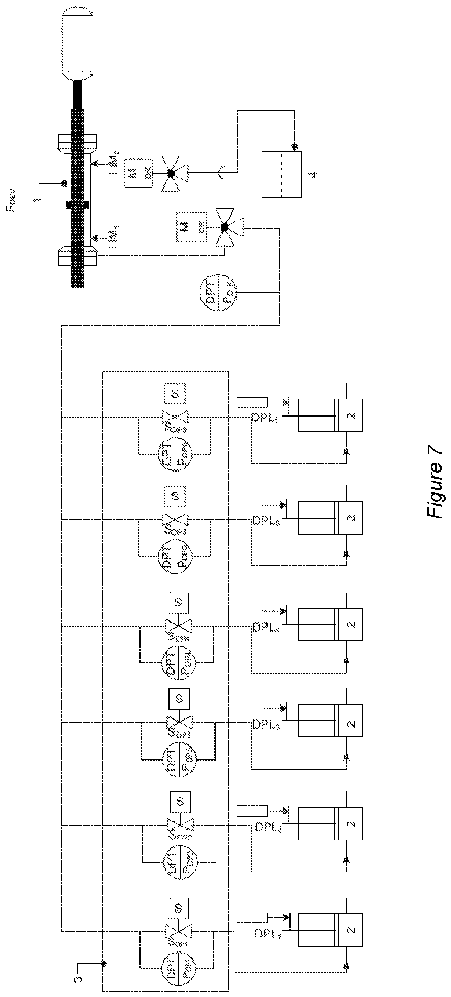

Figure 7 illustrates schematically the hydraulic system of the triaxial testing apparatus 100 according to the invention used to realize a displacement control required for generating the deviator voltage. In a conventional triaxial testing apparatus, to this end trapezoidal spindle or ball screw linear actuators are used. In a Bishop-Wesley cell device developed for stress field testing, the deviator voltage is generated by a piston that is driven by a stepper motor hydraulic pump. In the triaxial testing apparatus 100 according to the invention, the fluid flow required for the axial displacement is generated by a geared stepper motor driven hydraulic pump 1 with two working chambers. While one of the working chambers is working, the other one sucks oil from the oil tank to ensure continuous operation. Of course, other mechanism with a precision pump can also be used for the displacement control required for generating the deviator voltage.

The triaxial testing apparatus 100 according to the invention comprises a hydraulic actuator system with a closed-loop control for the displacement control necessary to generate the deviator voltage, whereby the feed rate of the test specimens in each of the measuring cells 10 can be controlled - mechanically in a displacement-controlled manner - by a single central pump and by means of appropriate sensing and control elements independently of each other and with sufficient accuracy.

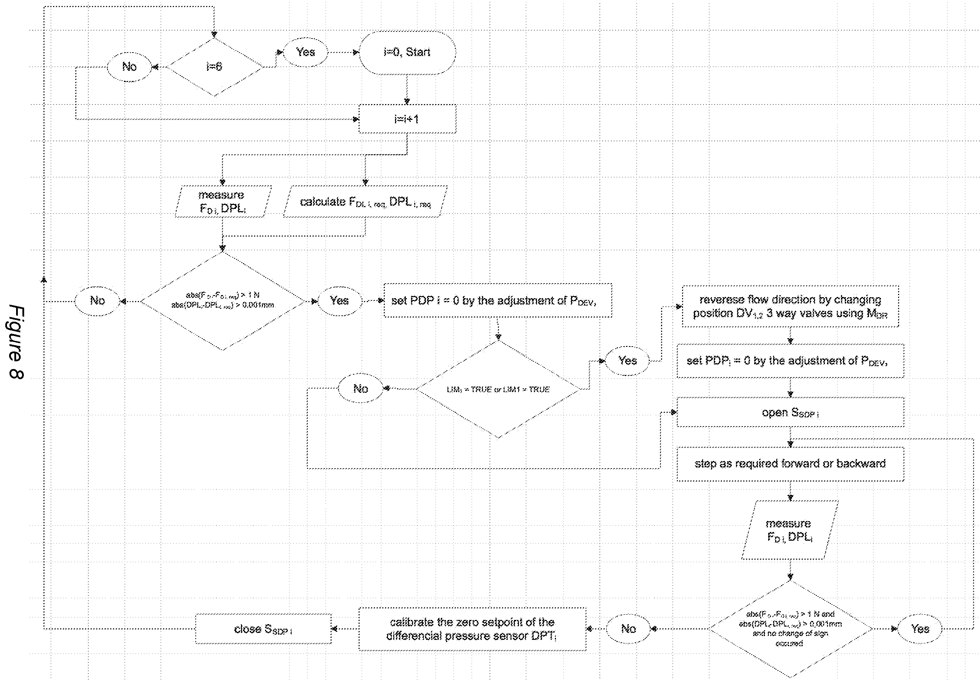

As shown in Figure 8, for the displacement control, based on the test routine, it is determined how much displacement (total compression or, in case of an optical measuring system, deformation) or deviator force is required at the given moment, and if a force or displacement can be measured that exceeds the calculated one with a value larger than a preset tolerance limit, intervention takes place. To this end, with the solenoid valve closed, a pressure equal to that of the measuring cell to be pressure controlled must be established by moving the pump in the appropriate direction. Completion of the pressure compensation is checked by nulling out the bidirectional differential pressure gauge associated with the measuring cell concerned. Once the pressure gets compensated, the solenoid valve is opened and the displacement- or force-controlled regulation is performed. After effecting the regulation, the differential pressure gauge signal is recorded; in the next iteration step this signal level will be the desired value for the compensation. By this technique, the control is made more precise by minimizing the effects of temperature variation and zero offset due to absolute pressure.

## Claims

1. A triaxial testing apparatus (100) comprising at least two measuring cells (10), each having a measuring volume, for performing triaxial measurements, each measuring volume being configured to receive a test specimen (7) subject to triaxial measurement, at least one control module (13) for controlling operation parameters in the at least two measuring cells (10) during the triaxial measurement, **characterized in that** the apparatus (100) further comprises a central hydraulic multiplexer unit interposed between the at least one control module (13) and the at least two measuring cells (10), said multiplexer unit being operatively connected with each of the measuring cells (10) and configured to introduce a gas medium from one single external central source (9) into each measuring volume as a medium for exerting hydrostatic load on the test specimen (7) in each respective measuring volume, **characterized in that** for controlling pore water pressure of the specimen the apparatus is provided with one single central pump connected to the lower end and the upper end of each of specimen by means of routing/switching mechanisms.

2. The triaxial testing apparatus (100) according to claim 1, **characterized in that** each of the at least two measuring cells (10) is configured as a pressure vessel made of a fibre reinforced material.

3. The triaxial testing apparatus (100) according to claim 1 or 2, **characterized in that** at least two optical cameras are arranged in each of the at least two measuring cells (10), said cameras configured to measure deformation of the test specimen (7) when the triaxial measurement is taken.

4. The triaxial testing apparatus (100) according to any one of claims 1-3, **characterized in that** the external source (9) of the medium for exerting hydrostatic load is a compressed air cylinder.

5. The triaxial testing apparatus (100) according to any one of claims 1-4, **characterized in that** each of the at least two measuring cells (10) is a conventional frame-based pressure cell or Bishop-Wesley triaxial cell.

## Patentansprüche

1. Triaxiale Prüfvorrichtung (100), umfassend mindestens zwei Messzellen (10), die jeweils ein Messvolumen aufweisen, zur Durchführung triaxialer Messungen, wobei jedes Messvolumen dazu eingerichtet ist, einen einer triaxialen Messung zu unterziehenden Prüfkörper (7) aufzunehmen, sowie mindestens ein Steuermodul (13) zum Steuern von Betriebsparametern in den mindestens zwei Messzellen (10) während der triaxialen Messung, **dadurch gekennzeichnet, dass** die Vorrichtung (100) ferner eine zentrale hydraulische Multiplexereinheit umfasst, die zwischen dem mindestens einen Steuermodul (13) und den mindestens zwei Messzellen (10) angeordnet ist, wobei die Multiplexereinheit mit jeder der Messzellen (10) wirkverbunden und dazu eingerichtet ist, ein gasförmiges Medium aus einer einzigen externen zentralen Quelle (9) in jedes Messvolumen als Medium zum Ausüben einer hydrostatischen Belastung auf den Prüfkörper (7) in dem jeweiligen Messvolumen einzuleiten, **dadurch gekennzeichnet, dass** die Vorrichtung zum Steuern des Porenwasserdrucks des Prüfkörpers mit einer einzigen zentralen Pumpe versehen ist, die mittels Leitungsführungs-/Umschaltmechanismen mit dem unteren Ende und dem oberen Ende jedes Prüfkörpers verbunden ist.

2. Triaxiale Prüfvorrichtung (100) nach Anspruch 1, **dadurch gekennzeichnet, dass** jede der mindestens zwei Messzellen (10) als ein aus einem faserverstärkten Material hergestellter Druckbehälter ausgebildet ist.

3. Triaxiale Prüfvorrichtung (100) nach Anspruch 1 oder 2, **dadurch gekennzeichnet, dass** in jeder der mindestens zwei Messzellen (10) mindestens zwei optische Kameras angeordnet sind, wobei die Kameras dazu eingerichtet sind, bei Durchführung der triaxialen Messung eine Verformung des Prüfkörpers (7) zu messen.

4. Triaxiale Prüfvorrichtung (100) nach einem der Ansprüche 1 bis 3, **dadurch gekennzeichnet, dass** die externe Quelle (9) des Mediums zum Ausüben der hydrostatischen Belastung eine Druckluftflasche ist.

5. Triaxiale Prüfvorrichtung (100) nach einem der Ansprüche 1 bis 4, **dadurch gekennzeichnet, dass** jede der mindestens zwei Messzellen (10) eine herkömmliche rahmenbasierte Druckzelle oder eine Bishop-Wesley-Triaxialzelle ist.

## Revendications

1. Appareil d'essai triaxial (100) comprenant au moins deux cellules de mesure (10), chacune présentant un volume de mesure, pour effectuer des mesures triaxiales, chaque volume de mesure étant configuré pour recevoir une éprouvette (7) soumise à une mesure triaxiale, ainsi qu'au moins un module de commande (13) destiné à commander des paramètres de fonctionnement dans les au moins deux cellules de mesure (10) pendant la mesure triaxiale, **caractérisé en ce que** l'appareil (100) comprend en outre une unité multiplexeur hydraulique centrale interposée entre l'au moins un module de commande (13) et les au moins deux cellules de mesure (10), ladite unité multiplexeur étant reliée fonctionnellement à chacune des cellules de mesure (10) et configurée pour introduire, dans chaque volume de mesure, un milieu gazeux provenant d'une seule source centrale externe (9), en tant que milieu destiné à exercer une charge hydrostatique sur l'éprouvette (7) dans chaque volume de mesure respectif, **caractérisé en ce que**, pour commander la pression de l'eau interstitielle de l'éprouvette, l'appareil est pourvu d'une seule pompe centrale reliée à l'extrémité inférieure et à l'extrémité supérieure de chaque éprouvette au moyen de mécanismes d'acheminement/de commutation.

2. Appareil d'essai triaxial (100) selon la revendication 1, **caractérisé en ce que** chacune des au moins deux cellules de mesure (10) est configurée sous la forme d'un récipient sous pression réalisé en un matériau renforcé de fibres.

3. Appareil d'essai triaxial (100) selon la revendication 1 ou 2, **caractérisé en ce qu'**au moins deux caméras optiques sont disposées dans chacune des au moins deux cellules de mesure (10), lesdites caméras étant configurées pour mesurer une déformation de l'éprouvette (7) lors de la réalisation de la mesure triaxiale.

4. Appareil d'essai triaxial (100) selon l'une quelconque des revendications 1 à 3, **caractérisé en ce que** la source externe (9) du milieu destiné à exercer la charge hydrostatique est une bouteille d'air comprimé.

5. Appareil d'essai triaxial (100) selon l'une quelconque des revendications 1 à 4, **caractérisé en ce que** chacune des au moins deux cellules de mesure (10) est une cellule de pression classique à bâti ou une cellule triaxiale de Bishop-Wesley.
